# FASCICULE DE BREVET EUROPEEN

(11) **EP 0 974 317 B1**
(45) Date de publication et mention de la délivrance du brevet: **03.03.2004**
(21) Numéro de dépôt: 99401870.3
(22) Date de dépôt: 23.07.1999
(51) Int. Cl.: A61F 2/36, A61F 2/46

(54) **Tige d'ancrage pour prothèse articulaire de hanche et jeu de telles tiges**
Verankerungsschaft für Hüftgelenkprothese und Satz von solchen Schäften
Anchoring shaft for hip joint prosthesis and set of such shafts

(30) Priorité: 24.07.1998 FR 9809504
(43) Date de publication de la demande: 26.01.2000
(73) Titulaire: BEJUI, Jacques, 69006 Lyon (FR); Carret, Jean-Paul, 69009 Lyon (FR); Chavane, Hervé, 69300 Caluire (FR); Pibarot, Vincent, 69007 Lyon (FR); Tayot, Olivier, 69002 Lyon (FR)
(72) Inventeur: BEJUI, Jacques, 69006 Lyon (FR); Carret, Jean-Paul, 69009 Lyon (FR); Chavane, Hervé, 69300 Caluire (FR); Pibarot, Vincent, 69007 Lyon (FR); Tayot, Olivier, 69002 Lyon (FR)
(74) Mandataire: Robert, Jean-Pierre

(56) Documents cités:
- EP-A- 0 477 113
- EP-A- 0 700 670
- FR-A- 2 625 433
- FR-A- 2 629 707
- FR-A- 2 636 837
- FR-A- 2 639 820
- FR-A- 2 666 984
- FR-A- 2 678 509
- FR-A- 2 729 292
- FR-A- 2 735 970
- GB-A- 2 281 213
- US-A- 5 792 143

## Description

La présente invention concerne une tige d'ancrage pour prothèse articulaire de hanche, ainsi qu'un jeu de telles tiges.

Les caractéristiques du préambule de la revendication 1 sont connues du document FR-A-2 666 984.

Une prothèse de hanche comprend principalement une tige d'ancrage destinée à être introduite dans le canal médullaire du fût fémoral pour y être scellée soit de façon naturelle par ostéointégration soit artificiellement au moyen d'un ciment et une tête articulaire sphérique se substituant à la tête fémorale naturelle pour être reçue dans la cavité cotyloïdienne de l'os iliaque correspondant du patient. La tige d'ancrage comporte une broche qui s'étend suivant un axe longitudinal et qui comprend d'une part une partie diaphysaire ayant une extrémité distale, et d'autre part une partie métaphysaire ayant une extrémité proximale. Les parties diaphysaire et métaphysaire assurent respectivement le guidage de la broche dans le fût fémoral et le maintien en position de la broche dans le fût fémoral.

La tête articulaire est raccordée à l'extrémité proximal de la partie métaphysaire de la broche par un col s'étendant en saillie d'une face terminale de cette extrémité proximale suivant un axe formant un angle (dit angle cervico-diaphysaire) avec l'axe longitudinal de la broche.

La partie métaphysaire de la broche présente couramment une section transversale en forme de trapèze. Cette forme assure un blocage efficace de la broche dans le canal médullaire du fût fémoral. Cependant, la broche ainsi formée est en contact avec l'os par des zones de largeur réduite correspondant aux arêtes du trapèze qui forment des zones de concentration de contraintes mécaniques. Il existe alors un risque de fragilisation de l'os et/ou de formation de cals osseux dans ces zones, ce qui peut engendrer des douleurs pour le patient.

Un but de l'invention est de fournir une tige d'ancrage assurant un maintien en position amélioré dans le fût fémoral tout en permettant une large répartition des contraintes mécaniques au niveau de la zone de contact entre la broche et l'os.

En vue de la réalisation de ce but, on prévoit, selon l'invention, une tige d'ancrage pour prothèse articulaire de hanche comprenant :
- une broche destinée à être insérée dans le fût fémoral, cette broche s'étendant suivant un axe longitudinal et comprenant une partie diaphysaire ayant une extrémité distale et une partie métaphysaire ayant une extrémité proximale et une partie intermédiaire entre les deux,
- un cône de support d'un élément sphérique d'articulation relié à la broche par un col de raccordement de manière à s'étendre en saillie d'une face terminale de l'extrémité proximale de la partie métaphysaire suivant un axe formant un angle dit angle cervico-diaphysaire avec l'axe longitudinal de la broche, la tige présentant un plan de symétrie contenant les axes de la broche et du cône et le profil de cette tige dans ce plan présentant un épaulement externe dans la zone inférieure de la partie métaphysaire,
ladite partie métaphysaire de la broche présentant en outre une section transversale en forme générale d'ogive ayant un extrémité étroite du côté de l'intrados de l'angle formé par les axes de la broche et du cône, l'angle au sommet dudit cône étant compris entre 5°30' et 6°, et en outre son diamètre maximal au voisinage du col étant de 12 millimètres tandis que son diamètre minimal est de 10 millimètres en extrémité, et l'axe du cône passant par le sommet de l'épaulement.

La partie méthaphysaire a de la sorte une section transversale dont la forme est analogue à celle de la section transversale du canal médullaire du fût fémoral. La partie métaphysaire est ainsi en contact avec l'os par la majeure partie de sa surface périphérique de sorte que les contraintes mécaniques sont largement réparties sur cette surface. En outre, cette forme améliore la stabilité de la broche dans le fût fémoral et assure un centrage progressif de la broche lors de son insertion dans le fût fémoral.

De préférence, le profil de la section transversale de la partie métaphysaire de la broche est émoussé. La répartition des contraintes est alors encore améliorée.

Selon un mode de réalisation préféré :
- le profil de la partie métaphysaire de la broche, dans un plan contenant l'axe longitudinal de la broche et perpendiculaire à son plan de symétrie, est divergent en direction de son extrémité proximale,
- le profil de l'extrémité distale de la partie diaphysaire, de la broche dans un plan contenant l'axe longitudinal de la broche et perpendiculaire à son plan de symétrie, est convergent en direction de cette extrémité,
- le profil de la partie intermédiaire de la broche entre les parties métaphysaire et diaphysaire, dans le plan contenant son axe longitudinal et perpendiculaire à son plan de symétrie, est sensiblement constant.

La partie métaphysaire assure un blocage progressif et homogène de la broche dans le fût fémoral, tandis que la fonction de guidage de la partie diaphysaire est renforcée et n'interfère pas avec la fonction de maintien en position assurée par la partie métaphysaire.

Par ailleurs, les tiges d'ancrage sont généralement organisées et commercialisées en jeux comprenant une série de tiges d'ancrage dites de première intention destinées à des patients recevant pour la première fois une prothèse articulaire de fémur et une série de tiges d'ancrage dites de reprise destinées en général à venir remplacer une tige de première intention implantée antérieurement. Les tiges de reprise ont des broches plus longues que celles des tiges de première intention afin de renforcer le fémur dans laquelle elles sont reçues. Chaque série rassemble des tiges d'ancrage de différentes tailles ayant des dimensions, notamment transversales, adaptées à différentes tailles de fémur.

Selon l'invention, on prévoit un jeu de tiges d'ancrage présentant au moins l'une des caractéristiques précitées, dans lequel le col des tiges de reprise est plus long que celui des tiges de première intention de l'ordre de 3,5 mm.

Cette surlongueur du col des tiges de reprise permet de compenser les distensions musculaires qui peuvent intervenir après la pose de la tige de première intention.

De préférence, les broches de la série de tiges de première intention sont homothétiques les unes des autres.

Selon des caractéristiques particulières, l'angle cervico-diaphysaire des tiges de première intention est sensiblement égal à 137°, et l'angle cervico-diaphysaire des tiges de reprise est sensiblement égal à 135°.

Du point de vue dimensionel, on pourra prévoir de préférence que :
- les broches de la série de tiges de première intention soient homothétiques les unes des autres ;
- entre les différentes tailles de la série de tiges de reprise, l'épaisseur de la partie métaphysaire des broches évolue linéairement,
- entre les différentes tailles de la série de tiges de reprise, l'épaisseur de la partie diaphysaire des broches évolue par paliers,
- entre les différentes tailles de la série de tiges de reprise, la largeur de la partie métaphysaire des broches 1 évolue linéairement,
- entre les différentes tailles de la série des tiges de reprise, la largeur de la partie diaphysaire des broches évolue par paliers.

D'autres caractéristiques et avantages de l'invention ressortiront à la lecture de la description qui suit d'un mode de réalisation particulier non limitatif de l'invention.

Il sera fait référence aux dessins annexés, parmi lesquels :
- la figure 1 est une vue de profil d'une tige d'ancrage selon l'invention,
- la figure 2 est une vue en coupe transversale selon la ligne II-II de la figure 1 de la partie métaphysaire de la broche, au voisinage de l'extrémité proximale,
- la figure 3 est une vue en coupe transversale selon la ligne III-III de la figure 1 de la partie métaphysaire de la broche ;
- la figure 4 est une vue en coupe transversale selon la ligne IV-IV de la figure 1 de la partie métaphysaire de la broche, au voisinage de la partie intermédiaire,
- la figure 5 est une vue en coupe transversale selon la ligne V-V de la figure 1 de la partie intermédiaire de la broche,
- la figure 6 est une vue en coupe transversale selon la ligne VI-VI de la figure 1 de l'extrémité distale de la partie diaphysaire,
- la figure 7 est une vue de face, selon la flèche VII de la figure 1, de la tige d'ancrage,
- la figure 8 est une vue de dessus de la tige d'ancrage,
- la figure 8A est un schéma illustrant une phase de mise en place de la tige dans le fût fémoral,
- la figure 9 est un diagramme mettant en évidence la variation, entre les différentes tailles des tiges de reprise de la largeur de la broche dans le plan P,
- la figure 10 est un diagramme représentant la variation, entre les différentes tailles des tiges de reprise, de l'épaisseur de la broche dans le plan P'.

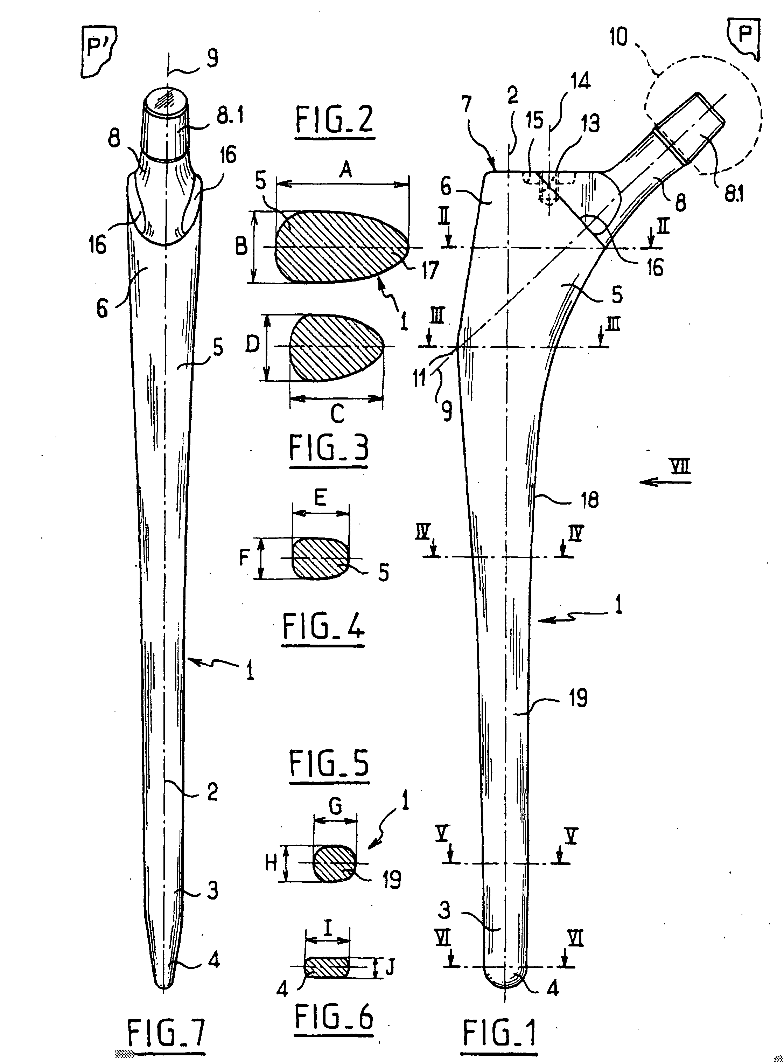

En référence à la figure 1, une tige d'ancrage selon l'invention comporte une broche, généralement désignée en 1, destinée à être insérée dans le canal médullaire du fût fémoral.

La broche 1 s'étend selon un axe longitudinal 2 et comprend une partie diaphysaire 3 ayant une extrémité distale 4, une partie intermédiaire 19, et une partie métaphysaire 5 ayant une extrémité proximale 6. La broche est en alliage de titane et peut être recouverte au moins sur sa partie métaphysaire d'un revêtement hydroxyapatite et/ou être superficiellement grenaillée afin de renforcer la résistance du scellement de la broche dans le fût fémoral.

L'extrémité proximale 6 de la partie métaphysaire 5 présente une face terminale 7 en saillie de laquelle s'étend un col de raccordement 8.

Le col 8 s'étend selon un axe formant un angle, dit cervico-diaphysaire, avec l'axe longitudinal 2 de la broche 1. Le col 8 a une portion d'extrémité conique 8.1 présentant un angle au sommet compris entre 5°30' et 6° et de préférence 5°45' d'angle pour l'emmanchement d'un élément sphérique 10 (représenté en pointillés) d'une tête articulaire destinée à être reçue dans la cavité cotyloïdienne de l'os iliaque du patient. La portion d'extrémité conique 8.1 du col 8 a un diamètre maximal égal à environ 12 mm du côté de la broche 1 et un diamètre minimal égal à environ 10 mm du côté de son extrémité libre. Le col 8 présente un décrochement à proximité de la face terminale 7 de la partie métaphysaire 5 du côté de l'extrados de l'angle cervico-diaphysaire. Cet embrèvement 10 ainsi que les dimensions précitées permettent l'obtention d'un important débattement angulaire de la tige d'ancrage par rapport à l'os iliaque, une fois la prothèse mise en place dans le corps du patient.

La tige d'ancrage présente un plan de symétrie p (le plan de la feuille à la figure 1) contenant l'axe longitudinal 2 de la broche 1 et l'axe 9 du cône 8.1 et a dans ce plan un profil général effilé vers l'extrémité distale. La tige d'ancrage présente en outre une courbure interne 18, du côté de l'intrados de l'angle cervico-diaphysaire, destinée à s'étendre dans le prolongement du cintre obturateur de l'os iliaque du patient.

En référence aux figures 1, 2, 3, 4 et 7, la partie métaphysaire 5 a un profil, dans le plan p, divergent en direction de son extrémité proximale 6 et sensiblement plus large que celui du reste de la broche 1. Dans un plan p' (le plan de la feuille à la figure 7), contenant l'axe longitudinal 2 et perpendiculaire au plan de symétrie p, le profil de la partie métaphysaire 5 diverge en direction de l'extrémité proximale 6 et est sensiblement plus large que celui du reste de la broche 1.

La partie métaphysaire 5 présente une section transversale en forme générale d'ogive ayant une extrémité 17 étroite du côté de l'intrados de l'angle cervico-diaphysaire (voir figures 2, 3 et 4). Le profil de cette section transversale est émoussé afin que la partie métaphysaire 5 ne présente pas d'arêtes risquant de former des zones de concentration de contraintes une fois la broche insérée dans le fût fémoral.

Le profil, dans les plans p et p', de la partie intermédiaire 19 s'étendant entre les parties métaphysaire 5 et diaphysaire 3 est sensiblement constant (voir figures 1 et 7). La partie intermédiaire 19 a une section transversale réduite sensiblement ovale (voir figure 5).

Le profil de la partie diaphysaire 3 de la broche 1, dans le plan p', est convergent en direction de l'extrémité distale 4, tandis que le profil de cette extrémité dans le plan p est sensiblement constant et prolonge le profil de la partie intermédiaire jusqu'à l'extrémité distale 4 au niveau de laquelle il se termine par une portion en arc de cercle (voir figure 1 et 7). Ces formes particulières de profil facilitent l'introduction de la broche dans le fût fémoral et améliore le guidage de la broche dans celui-ci. L'effilement de la partie diaphysaire 3 et éventuellement de la partie intermédiaire 19, et les dimensions transversales de celles-ci sont de préférence déterminées de façon que la partie diaphysaire 3 n'assure aucune fonction de maintien de la broche dans le fût fémoral qui risquerait d'interférer avec la fonction de maintien assurée par la partie métaphysaire et entrainerait une instabilité de la broche dans le fût fémoral.

En outre, la partie métaphysaire 5 comporte, sur la courbure externe de la broche s'étendant au niveau de l'extrados de l'angle cervico-diaphysaire, un épaulement externe 11 s'étendant dans le prolongement de l'axe 9 du col 8. Cet épaulement 11 permet la transmission au fût fémoral d'une partie importante des efforts exercés sur le col. Cet épaulement est légèrement arrondi pour une meilleure répartition des contraintes dans cette zone.

La tige d'ancrage selon l'invention est destinée à être insérée dans le fût fémoral et à être éventuellement extraite hors de celui-ci à l'aide d'un outil de montage/démontage. L'outil de montage/démontage comprend de manière générale un corps allongé pour sa préhension. Le corps allongé comprend une extrémité formant une tête de fixation de la tige d'ancrage sur l'outil en coopérant avec des moyens de la tête de fixation qui vont maintenant être décrits.

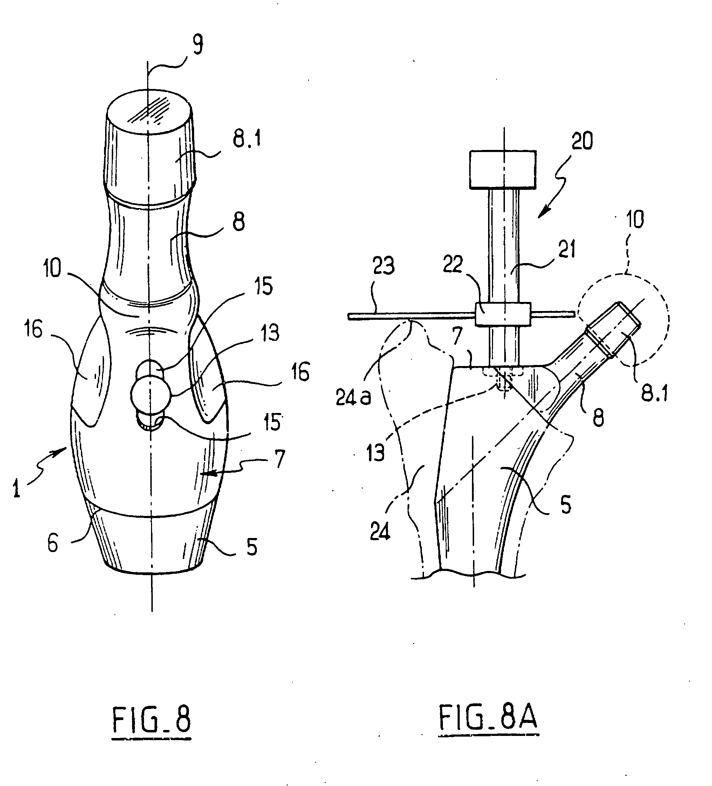

En référence aux figures 1, 8 et 8A, la face terminale 7 de la partie métaphysaire 5 présente un taraudage 13 s'étendant selon un axe 14 parallèle à l'axe longitudinal 2 de la broche 1.

Des lamages 15 s'étendent de part et d'autre du taraudage 13 symétriquement par rapport à l'axe 14 du taraudage 13.

Le taraudage 13 et les lamages 15 sont destinés à coopérer avec la tête de l'outil de montage/démontage permettant la préhension de la tige d'ancrage lors de son introduction dans le fût fémoral ou de son extraction hors de celui-ci. La tête de l'outil comprend par exemple une partie filetée destinée à être engagée dans le taraudage 13 et deux ergots escamotables commandés par une gachette et destinés à être reçus dans les lamages 15 une fois la partie filetée vissée dans le taraudage 13. On comprend qu'ainsi les lamages 15 forment des empreintes de blocage en rotation de l'outil par rapport à la tige d'ancrage autour de l'axe 14 du taraudage 13.

Lors des manipulations de la tige d'ancrage avec l'outil, la broche 1 peut ainsi être orientée autour de son axe longitudinal 2. Le taraudage 13 est de préférence légèrement décalé vers le col 8 par rapport à l'axe longitudinal 2 de la broche 1. Grâce à cette disposition particulière, le taraudage 13 de la broche une fois reçue dans le fût fémoral est écarté du grand trochanter qui pourrait constituer un obstacle au passage de l'outil. On pourrait également équiper la face terminale d'un doigt en saillie, aux lieu et place des lamages, ce doigt formant une butée pour au moins un ergot escamotable de la tête de l'outil et assurant ainsi le blocage en rotation de la tige d'ancrage par rapport à l'outil.

Le taraudage 13 possède une autre fonction : celle de recevoir un instrument tel que 20 représenté à la figure 8A qui permet de visualiser l'enfoncement de la tige dans le fût fémoral. Cet instrument comporte un support 21 dont une extrémité filetée vient se visser dans le taraudage 13. Ce support, en forme de tige, porte une bague 22 qui peut être ajustée en position le long de la tige 21 et qui peut tourner librement autour de celle-ci. La bague 22 reçoit à coulissement une aiguille 23 perpendiculaire à la tige 21 qui peut donc tourner avec la bague 22 autour de cette tige.

Ayant préalablement ajusté la position de la bague 22 sur la tige 21 de manière que l'aiguille 23 soit située au niveau du centre de la sphère 10, on visse l'instrument dans le taraudage 13 et on peut ainsi visualiser le niveau du centre de la sphère 10 par rapport au sommet 24a du grand trochanter 24 du fémur. On peut ainsi procéder à l'ajustement de l'enfoncement de la tige dans le fémur, jusqu'à ce que l'aiguille 23 frôle le sommet 24a du trochanter. On est alors certain que le centre de la sphère est dans sa position optimale.

Par ailleurs, les tiges d'ancrage selon l'invention peuvent être commercialisées sous forme de jeux comprenant une série de tiges de première intention et une série de tiges de reprise ayant des broches plus longues que celles des tiges de première intention afin de renforcer le fémur dans laquelle elles sont reçues. Les tiges d'ancrage de chaque série sont de tailles différentes et ont des dimensions, notamment transversales, différentes de manière à être adaptées à différentes tailles de fémur.

Les tiges de première intention présenteront avantageusement un angle cervico diaphysaire sensiblement égal à 137°. Cet angle particulier permet à la tige d'ancrage selon l'invention d'être adaptée à la majeure partie des patients devant être munis d'une prothèse articulaire de hanche.

Les broches des tiges de première intention sont de préférence homothétiques les unes des autres.

Les tiges de reprise ont préférentiellement un col plus long de l'ordre de 3,5 mm que celui des tiges de première intention. Les tiges de reprise présentent en outre un angle cervico-diaphysaire sensiblement égal à 135°. La surlongueur de 3,5 mm et cette valeur particulière de l'angle cervico-diaphysaire permettent de compenser les distensions musculaires ayant pû apparaître après la pose de la tige de première intention que la tige de reprise est destinée à remplacer.

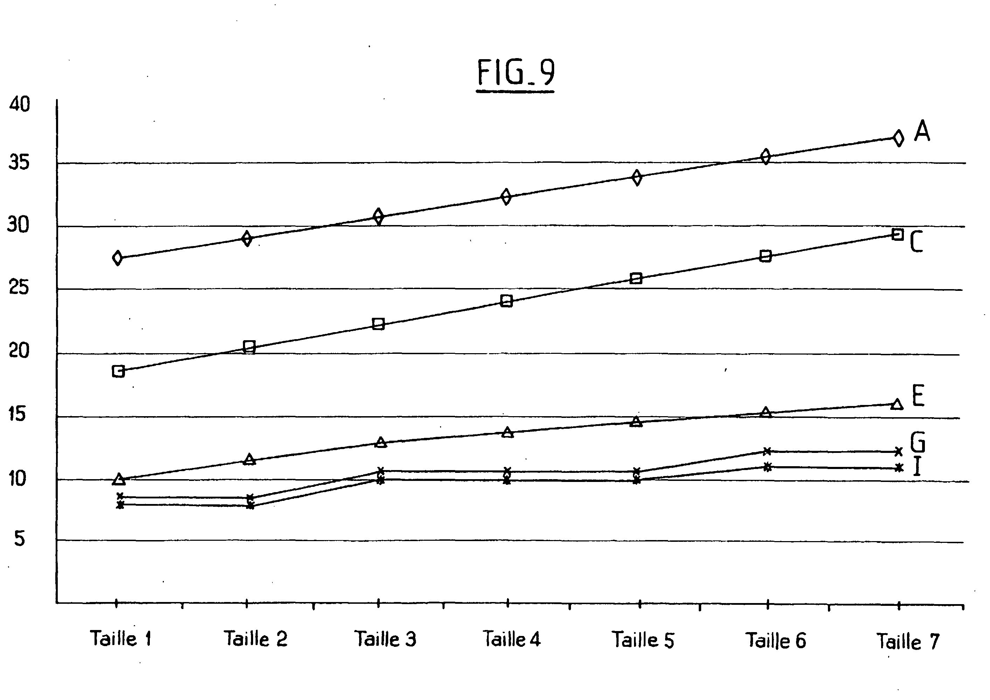

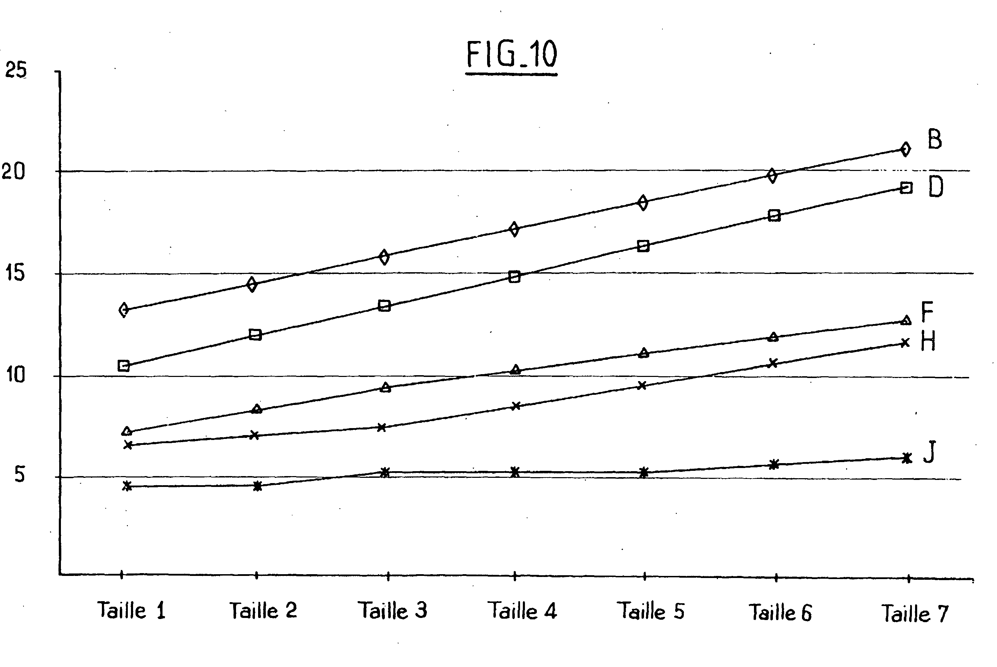

Les dimensions des tiges de reprise entre les différentes tailles de tiges d'une même série évoluent selon des lois de variation particulières qui sont représentées et définies par les figures 9 et 10.

A la figure 9, on a représenté les lois de variation en millimètre, les largeurs A, C, E, G, I des différentes sections transversales de la broche 1 représentées respectivement aux figures 2 à 6.

De même, à la figure 10, on a représenté les lois de variation des épaisseurs B, D, F, H, J des différentes sections de la broche 1 correspondant respectivement aux figures 2 à 6.

Au vu de ces graphiques, dont l'abscisse représente les tailles croissantes des tiges des reprises d'une même série et dont l'ordonnée représente la dimension considérée en millimètres (largeur à la figure 9 et épaisseur à la figure 10), on constate que les lois de variation de l'épaisseur et de la largeur de la broche 1 sont différentes suivant la section considérée. Ainsi, les sections II, III et IV correspondant aux figures 2, 3 et 4, sont situées dans la partie métaphysaire 5 et présentent une largeur A, C, E et une épaisseur B, D, F qui évoluent selon des lois de variation linéaire.

En revanche, la section VI correspondant à la figure 6, est située dans la partie diaphysaire 3 et présentent une largeur, I et une épaisseur J qui évoluent selon des lois de variation à palliers. On constate en particulier, pour la largeur I de la section 6, une constance entre là taille 1 et la taille 2, un saut d'augmentation entre la taille 2 et la taille 3, une constance entre la taille 3 et la taille 5, un nouveau saut d'augmentation entre la taille 5 et la taille 6 et une constance entre la taille 6 et la taille 7.

Bien entendu l'invention n'est pas limitée au mode de réalisation décrit et on peut y apporter des variantes de réalisation sans sortir du cadre de l'invention tel que défini par les revendications.

En particulier, on peut ménager sur la partie métaphysaire 5 de la broche des rainures interrompues en deça de la face terminale 7 de l'extrémité proximale 6. Ces rainures ont de préférence une section transversale arrondie. On pourrait également prévoir des rainures débouchant sur la face terminale 7.

## Revendications

1. Tige d'ancrage pour prothèse articulaire de hanche, comportant :
- une broche (1) destinée à être insérée dans le fût fémoral, cette broche s'étendant suivant un axe longitudinal (2) et comprenant une partie diaphysaire (3) ayant une extrémité distale (4) et une partie métaphysaire (5) ayant une extrémité proximale (6) et une partie intermédiaire (19) entre les deux,
- un cône (8.1) de support d'un élément sphérique (10) d'articulation relié à la broche par un col (8) de raccordement de manière à s'étendre en saillie d'une face terminale (7) de l'extrémité proximale (6) de la partie métaphysaire (5) suivant un axe (9) formant un angle dit angle cervico-diaphysaire avec l'axe longitudinal (2) de la broche (1)
la tige présentant un plan de symétrie (p) contenant les axes (2, 9) de la broche (1) et du cône (8.1) et le profil de cette tige dans ce plan présentant un épaulement (11) externe dans la zone inférieure de la partie métaphysaire (5),
**caractérisée en ce que** la partie métaphysaire (5) de la broche (1) présente une section transversale en forme générale d'ogive ayant un extrémité (17) étroite du côté de l'intrados de l'angle formé par les axes (2, 9) de la broche et du cône, **en ce que** l'angle au sommet du cône (8.1) est compris entre 5°30' et 6°, son diamètre maximal au voisinage du col (8) étant de 12 millimètres tandis que son diamètre minimal est de 10 millimètres en extrémité, et **en ce que** l'axe (9) du cône passe par le sommet de l'épaulement (11).

2. Tige d'ancrage selon la revendication précédente, **caractérisée en ce que** le profil de la partie métaphysaire (5) de la broche (1), dans un plan (p') contenant l'axe longitudinal (2) de la broche (1) et perpendiculaire à son plan de symétrie (p), est divergent en direction de son extrémité proximale (6).

3. Tige d'ancrage selon l'une des revendications précédentes, **caractérisée en ce que** le profil de l'extrémité distale (4) de la partie diaphysaire (3) de la broche (1), dans un plan (p') contenant l'axe longitudinal (2) de la broche (1) et perpendiculaire à son plan de symétrie (p), est convergent en direction de cette extrémité.

4. Tige d'ancrage selon l'une des revendications 2 et 3, **caractérisée en ce que** le profil de la partie intermédiaire (19) de la broche entre les parties métaphysaire (5) et diaphysaire (3), dans le plan (p') contenant son axe longitudinal (2) et perpendiculaire à son plan de symétrie, est sensiblement constant.

5. Tige d'ancrage selon l'une des revendications précédentes, **caractérisée en ce que** la partie métaphysaire (5) présente sur sa face terminale (7) un taraudage (13) sensiblement parallèle à l'axe longitudinal (2) de la broche (1) destiné à recevoir l'extrémité d'un support (21) d'une aiguille (23) d'affleurement du trochanter (24).

6. Jeu de tiges d'ancrage pour prothèse articulaire de hanche comprenant une série de tiges de première intention de différentes tailles et une série de tiges de reprise de différentes tailles selon l'une quelconque des revendications précédentes, **caractérisé en ce que** l'angle cervico-diaphysaire des tiges de première intention est sensiblement égal à 137°, **en ce que** l'angle cervico-diaphysaire des tiges de reprise est sensiblement égal à 135°, et **en ce que** le col (8) des tiges de reprise est plus long que celui des tiges de première intention de l'ordre de 3,5 mm.

7. Jeu de tiges selon la revendication 6, **caractérisé en ce que** les broches de la série de tiges de première intention sont homothétiques les unes des autres.

8. Jeu de tiges selon l'une des revendications 6 et 7, **caractérisé en ce que**, entre les différentes tailles de la série de tiges de reprise, l'épaisseur (B, D) de la partie métaphysaire (5) des broches (1) évolue linéairement, **en ce que** l'épaisseur (J) de la partie diaphysaire (3,4) des broches (1) évolue par paliers, **en ce que** la largeur (A,C) de la partie métaphysaire (5) des broches (1) évolue linéairement, et **en ce que** la largeur (I) de la partie diaphysaire (3, 4) des broches (1) évolue par paliers.

## Claims

1. An anchor rod for a hip joint prosthesis, the rod comprising:
a pin (1) for insertion in the shaft of the femur, the pin extending along a longitudinal axis (2) and comprising a diaphysial portion (3) having a distal end (4), and a metaphysial portion (5) having a proximal end (6), with an intermediate portion (19) between them,
a cone (8.1) for supporting a spherical joint element (10) connected to the pin via a connection neck (8) so as to project from a terminal face (7) of the proximal end (6) of the metaphysial portion (5) along an axis (9) forming a "cervico-diaphysial" angle with the longitudinal axis (2) of the pin (1),
the rod presenting a plane of symmetry (p) containing the axes (2, 9) of the pin (1) and of the cone (8.1) and the profile of the rod in this plane presenting an external shoulder (11) in the bottom zone of the metaphysial portion (5),
the anchor rod being **characterized in that** the metaphysial portion (5) of the pin (1) presents a cross-section that is generally bullet-shaped having a narrow end (17) beside the intrados of the angle formed by the axes (2, 9) of the pin and the cone, **in that** the angle at the apex of the cone (8.1) lies in the range 5°30' and 6°, its maximum diameter in the vicinity of the neck (8) being 12 millimeters (mm) while its minimum diameter is 10 mm at the end, and **in that** the axis (9) of the cone passes through the top of the shoulder (11).

2. An anchor rod according to the preceding claim, **characterized in that** the profile of the metaphysial portion (5) of the pin (1) in a plane (p') containing the longitudinal axis (2) of the pin (1) and perpendicular to its plane of symmetry (p), diverges towards its proximal end (6).

3. An anchor rod according to either preceding claim, **characterized in that** the profile of the distal end (4) of the diaphysial portion (3) of the pin (1) in a plane (p') containing the longitudinal axis (2) of the pin (1) and perpendicular to its axis of symmetry (p), converges towards said end.

4. An anchor rod according to claim 2 or claim 3, **characterized in that** the profile of the intermediate portion (19) of the pin between the metaphysial portion (5) and the diaphysial portion (3) in the plane (p') containing its longitudinal axis (2) and perpendicular to its plane of symmetry, is substantially constant.

5. An anchor rod according to any preceding claim, **characterized in that** the metaphysial portion (5) presents tapping (13) in its terminal face (7), the tapping extending substantially parallel to the longitudinal axis (2) of the pin (1) and serving to receive the end of a support (21) of a needle (23) for placing flush with the trochanter (24).

6. A set of anchor rods for a hip joint prosthesis, the set comprising a series of first intention rods of different sizes, and a series of repeat rods of different sizes, the rods in both series being according to any preceding claim, the set being **characterized in that** the cervico-diaphysial angle of the first intention rods is substantially equal to 137°, **in that** the cervico-diaphysial angle of the repeat rods is substantially equal to 135°, and **in that** the necks (8) of the repeat rods are longer than the necks of the first intention rods by about 3.5 mm.

7. A set of rods according to claim 6, **characterized in that** the pins of the series of first intention rods are geometrically similar to one another.

8. A set of rods according to claim 6 or claim 7, **characterized in that**, between the different sizes of the series of repeat rods, the thickness (B, D) of the metaphysial portion (5) of the pins (1) varies linearly, **in that** the thickness (J) of the diaphysial portion (3, 4) of the pins (1) varies in steps, **in that** the width (A, C) of the metaphysial portion (5) of the pins (1) varies linearly, and **in that** the width (I) of the diaphysial portion (3, 4) of the pins (1) varies in steps.

## Patentansprüche

1. Verankerungsschaft für eine Hüftgelenkprothese, umfassend:
- eine Spindel (1), die dazu vorgesehen ist, in den Oberschenkelknochenschaft eingesetzt zu werden und sich entlang einer Längsachse (2) erstreckt und einen Diaphysenabschnitt (3) mit einem distalen Ende (4) und einen Metaphysenabschnitt (5) mit einem proximalen Ende (6) sowie einen zwischen diesen beiden befindlichen intermediären Abschnitt (19) umfaßt,
- einen Konus (8.1) zum Halten eines sphärischen Gelenkelements (10), der über einen Verbindungshals (8) mit der Spindel derart verbunden ist, daß er über eine Endfläche (7) des proximalen Endes (6) des Metaphysenabschnitts hinaussteht und sich entlang einer Achse (9) erstreckt, die einen Winkel, den sogenannten Zerviko-Diaphysenwinkel, mit der Längsachse (2) der Spindel (1) bildet,
wobei der Schaft eine Symmetrieebene (p) hat, welche die Achsen (2, 9) der Spindel (1) und des Konus (8.1) enthält und das Profil des Schafts in dieser Ebene eine äußere Schulter (11) im unteren Bereich des Metaphysenabschnitts (5) umfaßt,
**dadurch gekennzeichnet, daß** der Querschnitt des Metaphysenabschnitt (5) der Spindel (1) im allgemeinen ogiv ist und auf der Innenseite des von den Achsen (2, 9) gebildeten Winkels der Spindel und des Konus ein schmales Ende (17) hat, daß der Winkel des Scheitelpunkts des Konus (8.1) zwischen 5°30' und 6° liegt, wobei sein größter Durchmesser angrenzend an den Hals (8) 12 Millimeter beträgt, während sein geringster Durchmesser am Ende 10 Millimeter beträgt, und daß die Achse (9) des Konus durch den Scheitelpunkt der Schulter (11) hindurch verläuft.

2. Verankerungsschaft nach dem vorhergehenden Anspruch, **dadurch gekennzeichnet, daß** das Profil des Metaphysenabschnitts (5) der Spindel (1) in einer Ebene (p'), welche die Längsachse (2) der Spindel (1) enthält und senkrecht zur Symmetrieebene (p) verläuft, in Richtung seines proximalen Endes (6) divergiert.

3. Verankerungsschaft nach einem der vorhergehenden Ansprüche, **dadurch gekennzeichnet, daß** das Profil des distalen Endes (4) des Diaphysenabschnitts (3) der Spindel (1) in einer Ebene (p'), welche die Längsachse (2) der Spindel (1) enthält und senkrecht zur Symmetrieebene (p) verläuft, in Richtung dieses Endes konvergiert.

4. Verankerungsschaft nach einem der Ansprüche 2 oder 3, **dadurch gekennzeichnet, daß** das Profil des intermediären Abschnitts (19) der Spindel zwischen dem Metaphysenabschnitt (5) und dem Diaphysenabschnitt (3) in der Ebene (p'), welche die Längsachse (2) enthält und senkrecht zur Symmetrieachse verläuft, im wesentlichen konstant ist.

5. Verankerungsschaft nach einem der vorhergehenden Ansprüche, **dadurch gekennzeichnet, daß** der Metaphysenabschnitt (5) auf seiner Endfläche (7) eine Gewindebohrung (13) aufweist, die im wesentlichen parallel zur Längsachse (2) der Spindel (1) verläuft und dazu vorgesehen ist, das Ende einer Halterung (21) einer Nadel (23) zur Abgleichung mit dem Trochanter (24) aufzunehmen.

6. Satz von Verankerungsschäften für eine Hüftgelenkprothese, umfassend eine Reihe von Schäften unterschiedlicher Größe für einen Primäreingriff und eine Reihe von Schäften unterschiedlicher Größe für einen weiteren Eingriff nach einem der vorhergehenden Ansprüche, **dadurch gekennzeichnet, daß** der Zerviko-Diaphysenwinkel der Schäfte für einen Primäreingriff im wesentlichen gleich 137° ist, der Zerviko-Diaphysenwinkel der Schäfte für den weiteren Eingriff im wesentlichen gleich 135° ist, und der Hals (8) der für den weiteren Eingriff eingesetzten Schäfte um etwa 3,5 mm länger ist als der Hals der für den Primäreingriff eingesetzten Schäfte.

7. Satz von Verankerungsschäften nach Anspruch 6, **dadurch gekennzeichnet, daß** die Spindeln der Reihe von Schäften für den Primäreingriff homothetisch sind.

8. Satz von Verankerungsschäften nach einem der Ansprüche 6 und 7, **dadurch gekennzeichnet, daß** sich die Dicke (B, D) des Metaphysenabschnitts (5) der Spindeln (1) zwischen den unterschiedlichen Größen der Reihe von Schäften für den weiteren Eingriff linear verändert, sich die Dicke (J) des Diaphysenabschnitts (3, 4) der Spindeln (1) stufenweise verändert, sich die Breite (A, C) des Metaphysenabschnitts (5) der Spindeln (1) linear verändert und sich die Breite (I) des Diaphysenabschnitts (3, 4) der Spindeln (1) stufenweise verändert.
